# EUROPÄISCHE PATENTANMELDUNG

(11) **EP 1 544 203 A1**
(43) Veröffentlichungstag der Anmeldung: **22.06.2005**
(21) Anmeldenummer: 04028926.6
(22) Anmeldetag: 07.12.2004
(51) Int. Cl.: C07D 495/04

(54) **3,4-Dioxythiophen-derivative**

(30) Priorität: 19.12.2003 DE 10359796
(71) Anmelder: H.C. Starck GmbH & Co. KG, 38642 Goslar (DE)
(72) Erfinder: Heuer, Helmut-Werner, Dr., 47829 Krefeld (DE); Wehrmann, Rolf, Dr., 47800 Krefeld (DE)
(74) Vertreter: Bramer-Weger, Elmar, Dr

(57) **Zusammenfassung**

Verfahren zur Herstellung von Verbindungen der Formel (I) 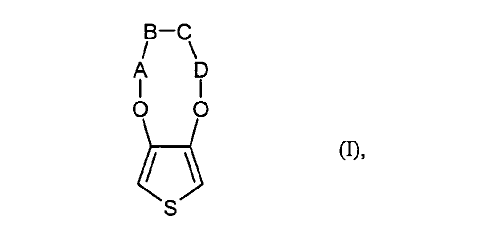 wobei A, B, C und D jeweils unabhängig voneinander für eine Bindung, gegebenenfalls substituiertes Alkylen, gegebenenfalls substituiertes Cycloalkylen, gegebenenfalls substituiertes Arylen oder (O-(CR¹R²)ₘ)ₓ, mit R¹ und R² = jeweils unabhängig voneinander Wasserstoff oder gegebenenfalls substituiertes Alkyl, m = eine ganze Zahl von 1 bis 10 und x = eine ganze Zahl von 1 bis 10, stehen, mit der Maßgabe, dass mindestens eine der Einheiten A, B, C oder D nicht für eine Bindung steht, durch Umsetzung einer Verbindung TosO-A-B-C-D-OTos mit einem geeigneten 3,4-Dihydroxythiophen oder dessen Alkalisalz und anschließende Verseifung und Decarboxylierung, und so erhältliche Verbindungen.

## Beschreibung

Die Erfindung betrifft ein Verfahren zur Herstellung von 3,4-Dioxythiophen-Derivaten durch Umsetzung von Ditosylaten oder Ditriflaten mit geeigneten 3,4-Dihydroxythiophenen oder deren Alkalisalze. Die Erfindung betrifft weiterhin so erhältliche Verbindungen, die als Bausteine für π-konjugierte Polymere eingesetzt werden können.

Die Verbindungsklasse der π-konjugierten Polymere war in den letzten Jahrzehnten Gegenstand zahlreicher Veröffentlichungen. Sie werden auch als leitfähige Polymere oder als synthetische Metalle bezeichnet.

Wegen der erheblichen Delokalisierung der π-Elektronen entlang der Hauptkette zeigen diese Polymere interessante (nichtlineare) optische Eigenschaften, und nach Oxidation oder Reduktion stellen sie gute elektrische Leiter dar. Dadurch können diese Verbindungen in verschiedenen praktischen Anwendungsgebieten eingesetzt werden, wie z.B. in der Datenspeicherung, der optischen Signalverarbeitung, der Unterdrückung elektromagnetischer Störungen (EMI) und der Sonnenenergieumwandlung, sowie in wiederaufladbaren Batterien, lichtemittierenden Dioden, Feldeffekttransistoren, Leiterplatten, Sensoren, Kondensatoren, elektrochromen Vorrichtungen und antistatischen Materialien.

Beispiele für bekannte π-konjugierte Polymere sind Polypyrrole, Polythiophene, Polyaniline, Polyacetylene, Polyphenylene und Poly(p-phenylen-vinylene).

Ein besonders wichtiges und technisch genutztes Polythiophen ist das Poly(ethylen-3,4-dioxythiophen) (Baytron® P), das in seiner oxidierten Form sehr hohe Leitfähigkeiten aufweist.

Aber auch andere Derivate des Poly(3,4-dioxythiophen)s sind interessante Verbindungen, da durch geeignete Wahl der Reste, die an die beiden Sauerstoffatome des 3,4-Dioxythiophens gebunden sind, beispielsweise die Löslichkeit in organischen Lösungsmitteln, die elektrochemischen Eigenschaften, sowie die Leitfähigkeit des Polymers gezielt beeinflusst werden können.

Problematisch für den Einsatz solcher Derivate des Poly(3,4-dioxythiophen)s ist, dass die benötigten Thiophen-Monomeren oft nur über aufwändige und mit geringen Ausbeuten verlaufende Synthesen zugänglich sind bzw. über gängige Methoden überhaupt nicht erhalten werden können.

Aufgabe der vorliegenden Erfindung ist daher die Bereitstellung einer einfachen, möglichst universell anwendbaren Synthese von 3,4-Dioxythiophen-Derivaten, mit der die gewünschten Produkte in guten Ausbeuten aus leicht zugänglichen Rohstoffen erhalten werden können.

Es wurde nun gefunden, dass auf einfache Weise eine Vielzahl von 3,4-Dioxythiophen-Derivaten hergestellt werden können, indem gut zugängliche 2,5-Dicarbalkoxy-3,4-dihydroxythiophene mit Ditosylaten oder Ditriflaten umgesetzt und anschließend hydrolysiert und decarboxyliert werden. Dieses Verfahren zeichnet sich insbesondere dadurch aus, dass eine Vielzahl von Ditosylaten zur Verfügung stehen und eingesetzt werden können, da diese sehr einfach aus den entsprechenden Diolen durch Umsetzung mit p-Toluolsulfonsäure erhalten werden können.

Gegenstand der Erfindung ist daher ein Verfahren zur Herstellung von Verbindungen der Formel (I) 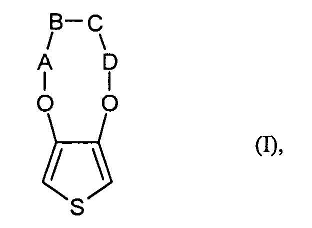 wobei
- A, B, C und D: jeweils unabhängig voneinander für eine Bindung, gegebenenfalls substituiertes Alkylen, gegebenenfalls substituiertes Cycloalkylen, gegebenenfalls substituiertes Arylen oder (-O-(CR¹R²)ₘ-)ₓ, bevorzugt gegebenenfalls substituiertes Cycloalkylen, gegebenenfalls substituiertes Arylen oder oder (-O-(CR¹R²)ₘ-)ₓ, mit
- R¹ und R²: ist jeweils unabhängig voneinander Wasserstoff oder gegebenenfalls substituiertes Alkyl, bevorzugt C₁-C₆-Alkyl,
- m: ist eine ganze Zahl von 1 bis 10 und
- x: ist eine ganze Zahl von 1 bis 10,
stehen, mit der Maßgabe, dass mindestens eine der Einheiten A, B, C oder D nicht für eine Bindung steht,
durch Umsetzung einer Verbindung der Formel (II)

TosO-A-B-C-D-OTos (II)

wobei
A, B, C und D die oben angegebene Bedeutung haben und
- Tos: für p-Toluolsulfonyl oder Trifluormethansulfonyl steht,
mit einem Thiophen der Formel (III) 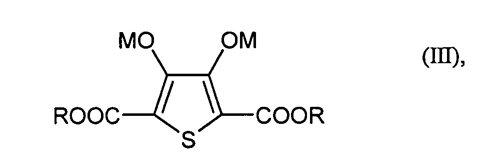 wobei
- R: für C₁-C₁₈-Alkyl und
- M: für H, Li, Na oder K steht,
und anschließende Verseifung und Decarboxylierung.

Unter substituiert ist hier und im Folgenden, wenn nicht anders erwähnt, insbesondere halogensubstituiert zu verstehen. Besonders bevorzugt ist eine Substitution mit F, Cl, Br oder insbesondere bevorzugt mit F.

A, B, C und D können jeweils eine Vielzahl unterschiedlicher Bedeutungen haben und so gemeinsam verschiedenste Einheiten ausbilden, so dass das erfindungsgemäße Verfahren äußerst vielseitig einsetzbar ist.

Beispielsweise können A, B, C und D gemeinsam für -(CR³R⁴)ₙ-(CH₂)ₒ-(CR⁵R⁶)ₚ- oder -(CH₂)_{q}-(CR⁷R⁸)ᵣ-(CH₂)ₛ- stehen, wobei R³, R⁴, R⁵, R⁶, R⁷ und R⁸ jeweils unabhängig voneinander für Wasserstoff, Halogen oder gegebenenfalls substituiertes C₁-C₁₀-Alkyl und n, o, p, q, r und s jeweils unabhängig voneinander für eine ganze Zahl von 1 bis 10 stehen.

Vorzugsweise stehen R³, R⁴, R⁵, R⁶, R⁷ und R⁸ jeweils unabhängig voneinander für Wasserstoff oder C₁-C₆-Alkyl.

Vorzugsweise stehen n, o, p, q, r und s jeweils unabhängig voneinander für eine ganze Zahl von 1 bis 5, besonders bevorzugt von 1 bis 3.

A, B, C und D können auch gemeinsam für gegebenenfalls substituiertes C₆-C₈-Cycloalkylen stehen, beispielsweise für Cyclohexylen oder Cyclooctylen.

In einer bevorzugten Ausführungsform stehen A, B, C und D gemeinsam für -((CR⁹R¹⁰)ₐ-O)_{b}-(CR¹¹R¹²)_{c}-, wobei R⁹, R¹⁰, R¹¹ und R¹² jeweils unabhängig voneinander für Wasserstoff oder gegebenenfalls substituiertes C₁-C₁₀-Alkyl, a und c jeweils unabhängig voneinander für eine ganze Zahl von 1 bis 10 und b für eine ganze Zahl von 1 bis 8 stehen.

Vorzugsweise stehen R⁹, R¹⁰, R¹¹ und R¹² jeweils unabhängig voneinander für Wasserstoff oder C₁-C₆-Alkyl, insbesondere bevorzugt stehen R⁹, R¹⁰, R¹¹ und R¹² jeweils für Wasserstoff.

a und c stehen vorzugsweise jeweils unabhängig voneinander für eine ganze Zahl von 1 bis 4, insbesondere bevorzugt für 2. b steht vorzugsweise für 1 oder 2.

Der besondere Vorteil des erfindungsgemäßen Verfahrens besteht darin, dass dieses Verfahren ohne die Anwendung von Phasentransferkatalysatoren auskommt, z.B. von quartären Ammoniumsalzen. Diese Phasentransferkatalysatoren sind kostspielige Verbindungen. Sie müssen nach der Reaktion durch zusätzliche Waschprozesse mittels wässriger Lösungen aus der Reaktionsmischung entfernt werden.

Die Entfernung von solche Phasentransferkatalysatoren enthaltenden Abwässern ist wiederum aufwändig und kostspielig.

Bevorzugt ist daher ein Verfahren, das ohne die Anwendungen von Phasentransferkatalysatoren oder ähnlichen Katalysatoren arbeitet.

In einer weiteren besonderen Ausführungsform des erfindungsgemäßen Verfahrens bilden A, B, C und D gemeinsam eine Einheit aus, die mindestens einen aromatischen Ring, vorzugsweise Phenylen oder Naphthylen, enthält.

Vorzugsweise stehen A, B, C und D gemeinsam für eine Einheit der Formel (IV) 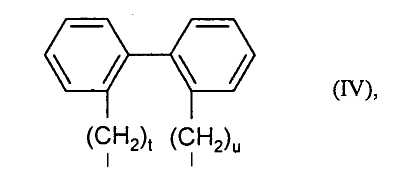 wobei
t und u jeweils unabhängig voneinander für eine ganze Zahl von 0 bis 5 stehen und
an die Phenylringe weitere aromatische Ringsysteme ankondensiert sein können. In letzterem Fall ist die Biphenyl-Einheit vorzugsweise durch eine Binaphthyl-Einheit ersetzt.

In einer weiteren bevorzugten Ausführungsform stehen A, B, C und D gemeinsam für -((CH₂)ᵥ-X-)_{y}-Ar-(X-(CH₂)_{w})_{z}-, wobei
- X: eine Bindung oder Sauerstoff und
- Ar: gegebenenfalls substituiertes Phenylen oder gegebenenfalls substituiertes Naphtylen bedeutet, und
- v und: w jeweils unabhängig voneinander für eine ganze Zahl von 1 bis 5 und
- y und z: jeweils unabhängig voneinander für eine ganze Zahl von 1 bis 10 stehen.

Erfindungsgemäß wird im ersten Reaktionsschritt ein Ditosylat oder Ditriflat mit einem Thiophen der Formel (III) 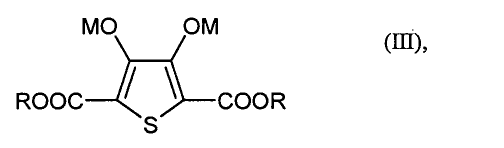 wobei
- R: für C₁-C₁₈-Alkyl und
- M: für H, Li, Na oder K steht,
umgesetzt.

Vorzugsweise steht R für C₁-C₁₀-Alkyl, insbesondere bevorzugt für Methyl, Ethyl oder n-Propyl, ganz besonders bevorzugt für Methyl.

M bedeutet vorzugsweise Wasserstoff.

Die Umsetzung des Thiophens der Formel III mit dem gewünschten Ditosylat oder Ditriflat der Formel II kann beispielsweise bei Normaldruck in dipolaren, aprotischen Lösemitteln in Gegenwart einer Base wie z.B. Kaliumcarbonat durchgeführt werden.

Vorzugsweise erfolgt die Umsetzung unter Schutzgasatmosphäre, beispielsweise unter Ar oder N₂.

Geeignete Lösungsmittel sind beispielsweise N-Methyl-2-pyrrolidon (NMP), Dimethylformamid, Dimethylacetamid, Dimethylsulfoxid oder hochsiedende Ketone. Bevorzugt wird als Lösungsmittel N-Methyl-2-pyrrolidon eingesetzt.

Die Umsetzung kann beispielsweise bei einer Temperatur von 80 bis 160°C, bevorzugt 90 bis 120°C durchgeführt werden.

Anschließend erfolgt eine Verseifung und eine Decarboxylierung.

Die Verseifung kann unter allgemein üblichen Bedingungen für eine solche Reaktion durchgeführt werden. Beispielsweise kann dabei so vorgegangen werden, dass in verdünnter Natron- oder Kalilauge erhitzt und anschließend mit Salz- oder Schwefelsäure neutralisiert wird.

Es kann sich ein Ansäuern anschließen. Das Ansäuern kann beispielsweise durch Zugabe von Säuren, insbesondere von Essigsäure bei Temperaturen von 0 bis 60°C erfolgen. Vorzugsweise wird soviel Säure zugegeben, dass sich bei der Temperatur der Umsetzung ein pH-Wert von 5-6 einstellt.

Auch die abschließende Decarboxylierung kann auf an und für sich bekannte Weise durchgeführt werden. Beispielsweise wird nach der Verseifung und gegebenenfalls Ansäuern die zu decarboxylierende Verbindung in Ethanolamin auf hohe Temperaturen, z. B. 160 bis 200°C, oder in einem dipolaren aprotischen Lösemittel wie Dimethylacetamid oder Dimethylsulfoxid in Gegenwart eines Katalysators wie basischem Kupfercarbonat oder Kupferchromit/Chinolin erhitzt.

Mit dem erfindungsgemäßen Verfahren lassen sich verschiedenste 3,4-Dioxythiophen-Derivate erhalten. Einige Verbindungen der Formel I, in der A, B, C und D gemeinsam eine Einheit ausbilden, die mindestens einen aromatischen Ring, vorzugsweise Benzylen oder Naphthylen, enthält, waren bislang nicht zugänglich und sind daher ebenfalls Gegenstand der Erfindung.

Die Erfindung betrifft demnach auch Verbindungen der Formel (I) 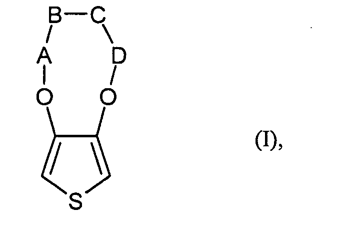 wobei A, B, C und D gemeinsam für eine Einheit der Formel (IV) 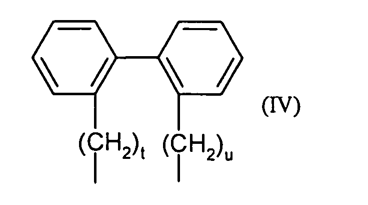 stehen, wobei
t und u jeweils unabhängig voneinander für eine ganze Zahl von 0 bis 5 stehen und an die Phenylringe weitere aromatische Ringsysteme ankondensiert sein können.

Die Erfindung betrifft auch Verbindungen der Formel (I) 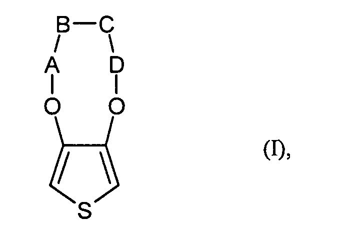 wobei A, B, C und D gemeinsam für -((CH₂)ᵥ-X-)_{y}-Ar-(X-(CH₂)_{w})_{z}- stehen, wobei
- X: eine Bindung oder Sauerstoff und
- Ar: gegebenenfalls substituiertes Phenylen oder gegebenenfalls substituiertes Naphtylen bedeutet, und

- v und w: jeweils unabhängig voneinander für eine ganze Zahl von 1 bis 5 und
- y und z: jeweils unabhängig voneinander für eine ganze Zahl von 1 bis 10 stehen.

Die erfindungsgemäß hergestellten 3,4-Dioxythiophen-Derivate lassen sich auf an sich bekannte Weise zu den entsprechenden Oligomeren und Polymeren umsetzen, die vielfältige Anwendung finden.

Dabei kann sowohl nur eine Verbindung der Formel I als Monomer eingesetzt werden, als auch ein Gemisch verschiedener Verbindungen, die unter die Definition der Formel I fallen. Es ist überdies möglich, neben einer oder mehrerer Verbindungen der Formel I auch weitere Thiophen-Derivate als Monomere zuzusetzen, insbesondere 3,4-Ethylendioxythiophen, das unter der Bezeichnung Baytron® M kommerziell erhältlich ist.

Die Polymerisation erfolgt entsprechend dem Vorgehen bei der Polymerisation bekannter Thiophen-Derivate. Sie kann beispielsweise oxidativ mit Oxidationsmitteln wie Eisen-III-chlorid oder anderen Eisen-III-Salzen, H₂O₂, Natrium- oder Kaliumperoxodisulfat, Kaliumdichromat, Kaliumpermanganat oder elektrochemisch erfolgen.

Die Erfindung wird nachfolgend anhand von Beispielen näher erläutert, wobei die Beispiele das Verständnis des erfindungsgemäßen Prinzips erleichtern sollen, und nicht als Einschränkung desselben zu verstehen sind.

### Beispiel 1

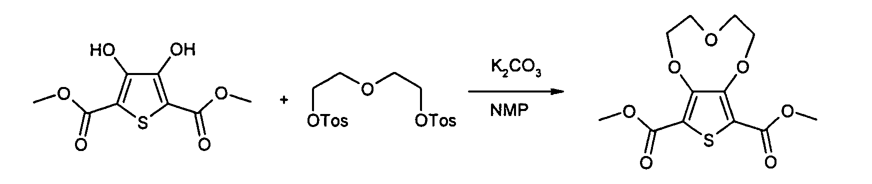

30,0 g (0,13 mol) 3,4-Dihydroxythiophen-1,2-dimethylester (Bayer AG, Leverkusen) und 53,88 g (0,13 mol) Diethylenglycol-(p-toluolsulfonat) (Fa. Alrich) wurden mit 44,22 g (0,32 mol) Kaliumcarbonat in 1500 ml N-Methylpyrrolidon (NMP) 18 Stunden lang bei 100°C gerührt. Der Reaktionsansatz wurde zur Aufarbeitung mit Wasser und Methylenchlorid versetzt und neutralgewaschen. Die organische Phase wurde abgetrennt und mit Natriumsulfat getrocknet. Nach dem Einengen der organischen Phase und Trocknung erhielt man beige-braunes Rohprodukt, welches als Hauptkomponente das gewünschte Produkt mit einer Masse von 302 enthielt (Analyse GC-MS).

Das Rohprodukt wurde durch Umkristallisation in Toluol gereinigt. Man erhielt 10,05 g (25,6 % der Theorie) eines hell-beige-farbenen Feststoffs.

Die Freisetzung der freien Thiophenverbinung kann in bekannter Weise durch Esterspaltung (Verseifen und Ansäuern) und anschließende Decarboxylierung, beispielsweise analog US-A 5,111,327 und EP 339 340 B1 erfolgen.

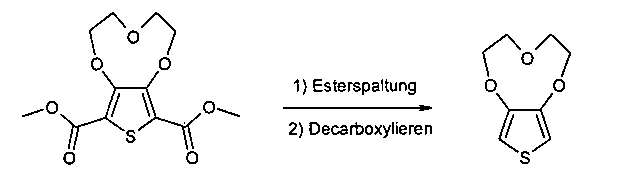

### Beispiel 2

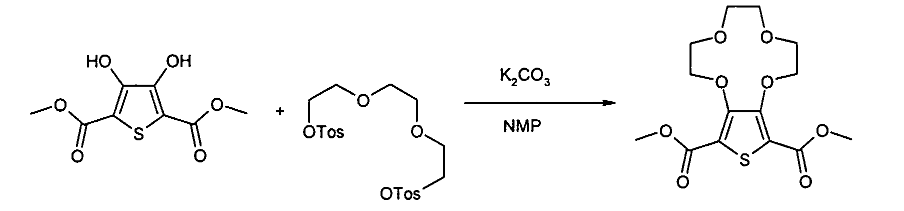

22,32 g (0,096 mol) 3,4-Dihydroxythiophen-1,2-dimethylester (Bayer AG, Leverkusen) und 45,85 g (0,1 mol) Triethylenglycol-(p-toluolsulfonat) (Fa. Alrich) wurden mit 34,55 g (0,25 mol) Kaliumcarbonat in 1500 ml N-Methylpyrrolidon 18 Stunden lang bei 100°C gerührt. Der Reaktionsansatz wurde zur Aufarbeitung mit Wasser und Methylenchlorid versetzt und neutralgewaschen. Die organische Phase wurde abgetrennt und mit Natriumsulfat getrocknet. Nach dem Einengen der organischen Phase und Trocknung erhielt man ein beige-braunes Rohprodukt, das in der massenspektrometrischen Untersuchung (GC-MS) als Hauptkomponente den Molekülpeak mit der Masse 346 zeigte.

Das Rohprodukt wurde durch Umkristallisation in Methanol gereinigt. Man erhielt 13,7 g (41,2 % der Theorie) eines beigen Feststoffs.

Die Freisetzung der freien Thiophenverbindung kann wiederum in bekannter Weise durch Esterspaltung (Verseifen und Ansäuern) und anschließende Decarboxylierung, beispielsweise analog US-A 5,111,327 und EP 339340 B1 erfolgen.

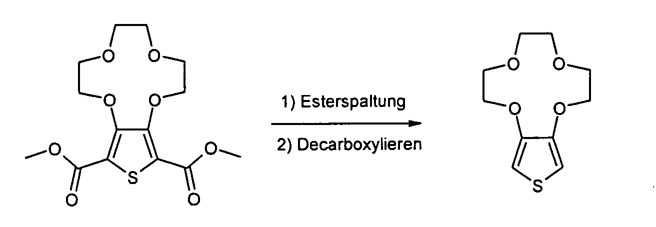

Auf analoge Art und Weise sind die folgenden Verbindungen zugänglich:

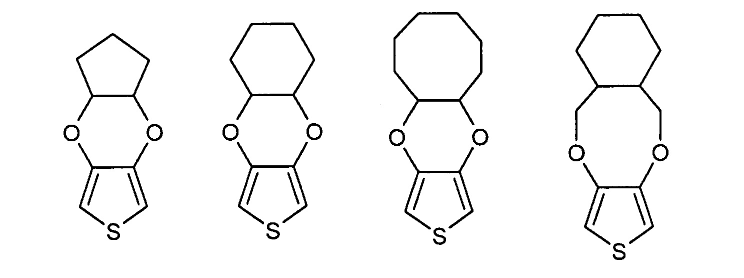

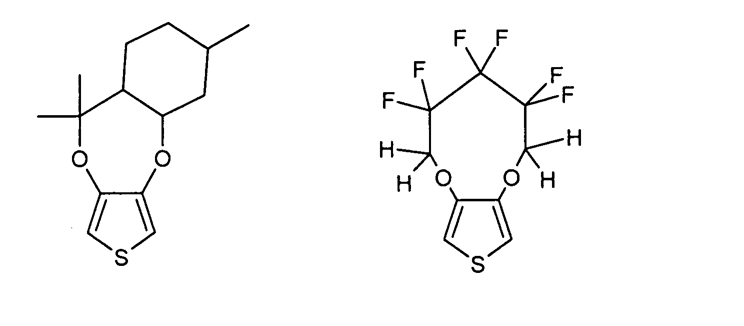

## Patentansprüche

1. Verfahren zur Herstellung von Verbindungen der Formel (I) 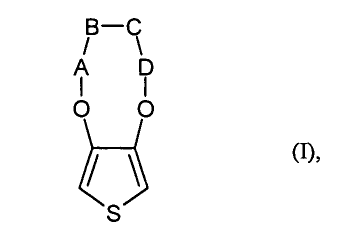 wobei
A, B, C und D jeweils unabhängig voneinander für eine Bindung, gegebenenfalls substituiertes Alkylen, gegebenenfalls substituiertes Cycloalkylen, gegebenenfalls substituiertes Arylen oder (O-(CR¹R²)ₘ)ₓ, bevorzugt gegebenenfalls substituiertes Cycloalkylen, gegebenenfalls substituiertes Arylen oder (O-(CR¹R²)ₘ)ₓ mit
R¹ und R² ist jeweils unabhängig voneinander Wasserstoff oder gegebenenfalls substituiertes Alkyl,
m ist eine ganze Zahl von 1 bis 10 und
x ist eine ganze Zahl von 1 bis 10
stehen, mit der Maßgabe, dass mindestens eine der Einheiten A, B, C oder D nicht für eine Bindung steht,
durch Umsetzung einer Verbindung der Formel (II)
TosO-A-B-C-D-OTos (II)
wobei
A, B, C und D die oben angegebene Bedeutung haben und
Tos für p-Toluolsulfonyl oder Trifluormethansulfonyl steht,
mit einem Thiophen der Formel (III) 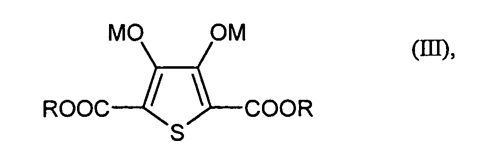 wobei
R für C₁-C₁₈-Alkyl und
M für H, Li, Na oder K steht,
und anschließende Verseifung und Decarboxylierung.

2. Verfahren nach Anspruch 1, **dadurch gekennzeichnet, dass** A, B, C und D gemeinsam für -(CR³R⁴)ₙ-(CH₂)ₒ-(CR⁵R⁶)ₚ- oder -(CH₂)_{q}-(CR⁷R⁸)ᵣ-(CH₂)ₛ- stehen, wobei R³, R⁴, R⁵, R⁶, R⁷ und R⁸ jeweils unabhängig voneinander für Wasserstoff, Halogen oder gegebenenfalls substituiertes C₁-C₁₀-Alkyl und n, o, p, q, r und s jeweils unabhängig voneinander für eine ganze Zahl von 1 bis 10 stehen.

3. Verfahren nach Anspruch 1, **dadurch gekennzeichnet, dass** A, B, C und D gemeinsam für C₆-C₈-Cycloalkylen stehen.

4. Verfahren nach Anspruch 1, **dadurch gekennzeichnet, dass** A, B, C und D gemeinsam für eine Einheit der Formel (IV) 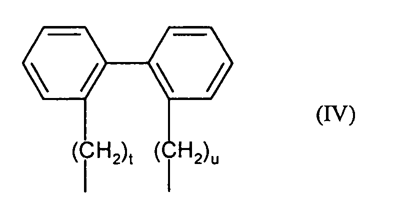 stehen, wobei
t und u jeweils unabhängig voneinander für eine ganze Zahl von 0 bis 5 stehen und
an die Phenylringe weitere aromatische Ringsysteme ankondensiert sein können.

5. Verfahren nach Anspruch 1, **dadurch gekennzeichnet, dass** A, B, C und D gemeinsam für -((CH₂)ᵥ-X-)_{y}-Ar-(X-(CH₂)_{w})_{z}- stehen, wobei
X eine Bindung oder Sauerstoff und
Ar gegebenenfalls substituiertes Phenylen oder gegebenenfalls substituiertes Naphthylen bedeutet, und
v und w jeweils unabhängig voneinander für eine ganze Zahl von 1 bis 5 und
y und z jeweils unabhängig voneinander für eine ganze Zahl von 1 bis 10 stehen.

6. Verfahren nach Anspruch 1, **dadurch gekennzeichnet, dass** A, B, C und D gemeinsam für -((CR⁹R¹⁰)ₐ-O)_{b}-(CR¹¹R¹²)_{c}- stehen, wobei R⁹, R¹⁰, R¹¹ und R¹² jeweils unabhängig voneinander für Wasserstoff oder gegebenenfalls substituiertes C₁-C₁₀-Alkyl, a und c jeweils unabhängig voneinander für eine ganze Zahl von 1 bis 10 und b für eine ganze Zahl von 1 bis 8 stehen.

7. Verfahren nach Anspruch 1, **dadurch gekennzeichnet, dass** es sich bei R um Methyl, Ethyl oder n-Propyl handelt.

8. Verbindung der Formel (I) 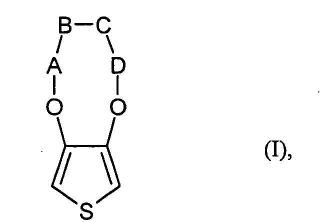 wobei
A, B, C und D gemeinsam für eine Einheit der Formel (IV) 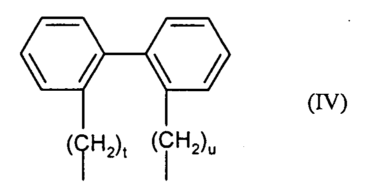 stehen, wobei
t und u jeweils unabhängig voneinander für eine ganze Zahl von 0 bis 5 stehen und
an die Phenylringe weitere aromatische Ringsysteme ankondensiert sein können.

9. Verbindung der Formel (I) 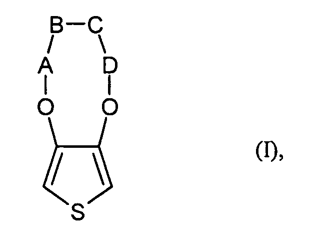 wobei
A, B, C und D gemeinsam für -((CH₂)ᵥ-X-)_{y}-Ar-(X-(CH₂)_{w})_{z}- stehen, wobei
X eine Bindung oder Sauerstoff und
Ar gegebenenfalls substituiertes Phenylen oder gegebenenfalls substituiertes Naphtylen bedeutet, und
v und w jeweils unabhängig voneinander für eine ganze Zahl von 1 bis 5 und
y und z jeweils unabhängig voneinander für eine ganze Zahl von 1 bis 10 stehen.
